# EUROPEAN PATENT APPLICATION

(11) **EP 4 755 195 A1**
(43) Date of publication of application: **10.06.2026**
(21) Application number: 24217705.3
(22) Date of filing: 05.12.2024
(51) Int. Cl.: A23G 3/36, A23G 3/50, A23P 10/20, A61K 9/20, A61K 31/592, A61K 33/30

(54) **COMESTIBLE COMPOSITION FOR CHEWABLE TABLETS AND PREPARATION METHOD OF TABLETS**

(71) Applicant: Haleon US Holdings LLC, Wilmington, DE 19808 (US)
(72) Inventor: Sankaranarayanan, Anand, 501401 Hyderabad (IN)
(74) Representative: Haleon Patent Department

(57) **Abstract**

Disclosed a composition, comprising an intragranular component and an extragranular component, the intragranular component comprising a diluent and a binder, wherein the diluent comprises 40 wt% or more of a first diluent based on the total weight of the diluent, the first diluent comprises one or more selected from the group consisting of : calcium carbonate, tricalcium phosphate, magnesium carbonate, and mixtures thereof, and the binder comprises a saccharide, an emulsifier and a fat. Further disclosed are a tablet prepared from materials comprising the composition, and a method for preparing a tablet.

## Description

### INVENTION FIELD

The present invention relates to comestible products, and particularly to such products with chewy mouth feel.

### BACKGROUND

Mouth feel is an important factor in evaluating comestible products. Chewy mouth feel becomes popular due to the special texture. Currently, conventional confectionary methods are used to prepare soft chewable products, but these methods are cumbersome, time consuming, tedious and process critical. Therefore, it is desirable to develop chewable products with a time-saving and cost-efficient preparation method.

### SUMMARY

The present inventor has conducted extensive research and developed a composition, from which a soft chewable tablet can be prepared, and the preparation method of the chewable tablet is time-saving and cost-efficient.

The present invention provides a composition, comprising an intragranular component and an extragranular component, the intragranular component comprising a diluent and a binder, wherein the diluent comprises 40 wt% or more of a first diluent based on the total weight of the diluent, the first diluent comprises one or more selected from the group consisting of: calcium carbonate, tricalcium phosphate, magnesium carbonate, and mixtures thereof, and the binder comprises a saccharide, an emulsifier and a fat.

Suitably, the saccharide comprises one or more selected from the group consisting of: liquid glucose, sorbitol solution, maltitol syrup, corn syrup, maple syrup, brown rice syrup, liquid maltrin, a solution of fructo oligosaccharide powder, and mixtures thereof.

Suitably, the emulsifier comprises one or more selected from the group consisting of: ammonium phosphatide, a lecithin, a polyglycerol polyricinoleate, an acetate of monodiglyceride, an acetate of diglyceride, and mixtures thereof.

Suitably, the fat comprises one or more selected from the group consisting of: a vegetable fat, a hydrogenated fat/oil, milk butter, and mixtures thereof.

Suitably, the binder comprises 85 wt% or more of the saccharide based on the total weight of the binder.

Suitably, the binder comprises 5 wt% or less of the emulsifier based on the total weight of the binder.

Suitably, the binder comprises 10 wt% or less of the fat based on the total weight of the binder

In some embodiments, the binder comprises 90-94 wt% of the saccharide, 0.5-2 wt% of the emulsifier, and 5-10 wt% of the fat, based on the total weight of the binder.

Suitably, the diluent further comprises a second diluent selected from the group consisting of: sucrose, maltitol, starch, and mixtures thereof.

Suitably, the weight ratio of the diluent to the binder comprises 2:1 to 0.6:1.

In some embodiments, the extragranular component comprises a lubricant, a sweetener, and/or an active ingredient.

Suitably, the lubricant comprises magnesium stearate, and/or silicon dioxide.

Suitably, the active ingredient comprises one of more of selected from the group consisting of: vitamins, minerals, supplements, active pharmaceutical ingredients, natural ingredients, enzymes, fibers, probiotics, prebiotics, postbiotics and mixtures thereof.

Suitably, the active ingredient comprises one of more of selected from the group consisting of: calcium, magnesium, vitamin D2, zinc citrate, UCII (Collagen), vitamin B, melatonin, L-theanine, probiotics, pepper, ginger, cumin, black salt, and mixtures thereof.

Suitably, the weight ratio of the intragranular component to the extragranular component comprises 1.5:1 to 5:1.

Further, the present invention provides a tablet prepared from materials comprising the above composition.

In some embodiments, the present invention provides a tablet prepared from the above composition.

Suitably, the tablet is chewable.

Suitably, the tablet has a weight of 800 mg, 900 mg, 1000 mg, 1100 mg, 1200 mg, 1300 mg, 1400 mg, 1500 mg, 1600 mg, 1700 mg, 1800 mg, 1900 mg, 2000 mg, 2100 mg, 2200 mg, 2300 mg, 2400 mg, or 2500 mg.

Suitably, the materials comprising the above composition have a bulk density of 0.5 to 1 g/ml.

In some embodiments, the above composition has a bulk density of 0.5 to 1 g/ml.

Still further, the present invention provides a method for preparing a tablet from materials comprising the composition, comprising:
(1) mixing the diluent and the binder by adding the binder into the diluent;
(2) granulating the mixture obtained from Step (1) to form granules;
(3) mixing the extragranular component with the granules obtained in Step (2); and
(4) compressing.

The features and advantages of the present invention will be particularly presented in detail in the following description of the embodiments.

### DETAILED DISCRIPTION

In the present application, unless expressly stated otherwise, the use of a singular comprises a plural and the use of a plural comprises a singular. For example, although "a" diluent is referred to herein, one or more of the species can be used.

In the present application, the terms "include," "comprise," and "contain" or the like are not intended to limit the invention to exclude any variations or additions. In the present application, unless expressly stated otherwise, the use of "or" means "and/or", even though "and/or" can be expressly used in some cases.

Additionally, it should be understood that any numerical range described herein is intended to encompass all the sub-ranges contained therein. For example, a range of "1 to 10" is intended to comprise all the sub-ranges between the listed minimum value of 1 and the listed maximum value of 10 (including the endpoints), namely, all the sub-ranges having a minimum value equal to or great than 1 and a maximum value equal to or less than 10.

Except those in the examples or otherwise expressly stated, it should be understood that all numerical values representing amounts of components used in the specification and claims can be varied by the term "about" in all cases. Accordingly, the numerical parameters set forth in the following description and claims are approximations that can vary depending upon desired properties to be obtained by the present invention, unless otherwise indicated to be contrary. At the least, it is intended not to limit the application of the doctrine of equivalent to the scope of the claims, and each numerical parameter should at least be construed in terms of its number of significant digits and by applying ordinary rounding.

Although the numerical ranges and parameters describing the broad scope of the present invention are approximations, the numerical values listed in the particular Examples should be reported as precisely as possible. However, any one value inherently has a certain error, which is an inevitable consequence of standard deviation found in its corresponding measurement method.

As recited above, the present invention refers to a composition, comprising an intragranular component and an extragranular component, the intragranular component comprising a diluent and a binder, wherein the diluent comprises 40 wt% or more of a first diluent based on the total weight of the diluent, the first diluent comprises one or more selected from the group consisting of: calcium carbonate, tricalcium phosphate, magnesium carbonate, and mixtures thereof, and the binder comprises a saccharide, an emulsifier and a fat.

The composition according to the present invention can be used to prepare a tablet. As used herein, the term "tablet" refers to a solid dosage form which can be prepared from compression. The tablet can be a pharmaceutical tablet, which comprises an active pharmaceutical ingredient. The tablet can be a non-pharmaceutical tablet, which does not comprise any active pharmaceutical ingredient. The tablet can be made in a variety of shapes, including round, or elongated, such as flattened ovoid or cylindrical shapes.

As used herein, the term "intragranular component" refers to the intragranular part of a tablet and comprises the compounds or ingredients in the composition added prior to granule formation/granulation. As used herein, the term "extragranular component" refers to the extragranular part of a tablet and comprises the compounds or ingredients in the composition added after granule formation/granulation and before compression.

As used herein, the "diluent", also referred to as "base" or "filler", increases bulk of the composition to facilitate compression or create sufficient bulk for homogenous blend for tablet formulations. Suitably, the diluent used in the present invention can be a dry diluent.

As used herein, the "binder" is used to hold the ingredients together in a composition. Suitably, the binder used in the present invention can be a liquid binder, e.g., a liquid form of a compound with no solvent, such as a melted compound, a compound dissolved/dispersed in a solvent, etc.

In the composition according to the present invention, the diluent can comprise 40 to 100 wt% of a first diluent based on the total weight of the diluent. For example, the diluent can comprise 45 wt% or more, 50 wt% or more, 55 wt% or more, 60 wt% or more, 65 wt% or more, 70 wt% or more, 75 wt% or more, 80 wt% or more, 85 wt% or more, 90 wt% or more, 95 wt% or more, or even 100 wt% of a first diluent based on the total weight of the diluent.

In the composition of the present invention, the binder can further comprise a second diluent, which is different from the first diluent. The second diluent can be a dry diluent suitable for use in a tablet. Suitably, the second diluent can comprise, but not be limited to, sucrose maltitol, starch, and mixtures thereof.

In the composition of the present invention, the saccharide of the binder can comprise one or more selected from the group consisting of: liquid glucose, sorbitol solution, maltitol syrup, corn syrup, maple syrup, brown rice syrup, liquid maltrin, a solution of fructo oligosaccharide powder, and mixtures thereof. Suitably, the sorbitol solution can comprise a sorbitol 70% solution. Suitably, the solution of fructo oligosaccharide powder can comprise fructo oligosaccharide powder dissolved in water. For example, the solution of fructo oligosaccharide powder can comprise fructo oligosaccharide powder dissolved in 20 wt% of water on the basis of the fructo oligosaccharide powder.

Based on the total weight of the binder, the saccharide can be present in an amount of 85 wt% or more. Suitably, the saccharide can be present in an amount of 85 wt% or more, 86 wt% or more, 87 wt% or more, 88 wt% or more, 89 wt% or more, 90 wt% or more, or 91 wt% or more, and/or 96 wt% or less, 95 wt% or less, 94 wt% or less, 93 wt% or less, or 92 wt% or less, based on the total weight of the binder. For example, the saccharide can be present in an amount of 85 to 96 wt%, 88 to 95 wt%, 90 to 94 wt%, within any range using any of the above values as endpoints.

In the composition of the present invention, the emulsifier of the binder can comprise one or more selected from the group consisting of: ammoium phosphatide, a lecithin, a polyglycerol polyricinoleate, an acetate of monodiglyceride, an acetate of diglyceride, and mixtures thereof. Suitably, the lecithin can be derived from a soya and/or a sunflower. Suitably, the polyglycerol polyricinoleate can be derived from a castor and/or a soya.

Based on the total weight of the binder, the emulsifier can be present in an amount of 5 wt% or less. Suitably, the emulsifier can be present in an amount of 5 wt% or less, 4 wt% or less, 3 wt% or less, 2 wt% or less, 1.9 wt% or less, 1.8 wt% or less, 1.7 wt% or less, 1.6 wt% or less, 1.5 wt% or less, or 1.4 wt% or less, and/or 0.4 wt% or more, 0.5 wt% or more, 0.6 wt% or more, 0.7 wt% or more, 0.8 wt% or more, 0.9 wt% or more, 1 wt% or more, 1.1 wt% or more, 1.2 wt% or more, 1.3 wt% or more, or 1.4 wt% or more, based on the total weight of the binder. For example, the emulsifier can be present in an amount of 0.4 to 5 wt%, 0.5 to 2 wt%, 0.6 to 1.3 wt%, within any range using any of the above values as endpoints.

In the composition of the present invention, the fat of the binder can comprise one or more selected from the group consisting of: a vegetable fat, a hydrogeneated fat/oil, milk butter, and mixtures thereof.

Based on the total weight of the binder, the fat can be present in an amount of 10 wt% or less. Suitably, the fat can be present in an amount of 10 wt% or less, 9 wt% or less, 8 wt% or less, or 7 wt% or less, and/or 1 wt% or more, 2 wt% or more, 3 wt% or more, 4 wt% or more, 5 wt% or more, or 6 wt% or more, based on the total weight of the binder. For example, the fat can be present in an amount of 1 to 10 wt%, 2 to 9 wt%, 5 to 10 wt%, 5 to 8 wt%, within any range using any of the above values as endpoints.

In some embodiments, the binder can comprise 90 to 94 wt% of the saccharide, 0.5 to 2 wt% of the emulsifier, and 5 to 8 wt% of the fat, based on the total weight of the binder.

Suitably, the weight ratio of the diluent to the binder in the composition can comprise 2:1 to 0.6:1. Suitably, the weight ratio of the diluent to the binder in the composition can be 2:1 or less, 1.9:1 or less, 1.8:1 or less, 1.7:1 or less, 1.6:1 or less, 1.5:1 or less, 1.4:1 or less, 1.3:1 or less, 1.2:1 or less, and/or 0.6:1 or more, 0.7:1 or more, 0.8:1 or more, 0.9:1 or more, 1:1 or more, or 1.1:1 or more. For example, the weight ratio of the diluent to the binder in the composition can be 2:1 to 0.6:1, 1.5:1 to 0.8:1, 1.3:1 to 0.9:1, or within any range using any of the above ratios as endpoints.

In the composition of the present invention, the extragranular component can comprise a lubricant, a sweetener, and/or an active ingredient.

Examples of lubricant can comprise, but not be limited to, stearic acid, magnesium stearate, calcium hydroxide, talc, sodium stearyl fumarate, a hydrocarbon such as mineral oil, higher fatty acids and their alkali-metal and alkaline earth metal salts, such as aluminum, calcium, magnesium, zinc, stearic acid, sodium stearates, glycerol, talc, waxes, boric acid, sodium benzoate, sodium acetate, sodium chloride, leucine, a polyethylene glycol (e.g., PEG-4000) or a methoxypolyethylene glycol such as Carbowax^{™}, sodium oleate, sodium benzoate, glyceryl behenate, polyethylene glycol, magnesium or sodium lauryl sulfate, colloidal silica such as Syloid^{™}, Cab-O-Sil^{®}, silicone dioxide, and the like. Suitably, the lubricant can comprise magnesium stearate, and/or silicon dioxide. For example, the lubricant can magnesium stearate and/or silicon dioxide.

Based on the total weight of the composition, the lubricant can be present in an amount of 0.5 to 3 wt%. Suitably, the lubricant can be present in an amount of 0.5 wt% or more, 0.7 wt% or more, 0.9 wt% or more, 1.1 wt% or more, 1.2 wt% or more, 1.3 wt% or more, 1.4 wt% or more, or 1.5 wt% or more, and/or 3 wt% or less, 2.8 wt% or less, 2.6 wt% or less, 2.4 wt% or less, 2.2 wt% or less, 2 wt% or less, 1.9 wt% or less, 1.8 wt% or less, 1.7 wt% or less, 1.6 or less, based on the total weight of the composition.

Suitably, the active ingredient can comprise, but not be limited to, one of more of selected from the group consisting of: vitamin, minerals, supplements, collagen, active pharmaceutical ingredient, natural ingredients, probiotic, fibers, prebiotic, postbiotic and mixtures thereof. Suitably, the active ingredient can comprise one of more of selected from the group consisting of: calcium, magnesium, vitamin D, zinc citrate, UCII (Collagen), vitamin B, melatonin, L-theanine, probiotics, pepper, ginger, cumin, black salt, and mixtures thereof. Suitably, the active ingredient can comprise an active pharmaceutical ingredient

The active ingredient can be present in an effective amount in the composition. As used herein, the term "effective amount" or "therapeutically effective amount," refers to a sufficient amount of an active ingredient being administered that would be expected to relieve to some extent one or more of the symptoms of the disease or condition being treated.

In some embodiment, the active ingredient can be present in the extragranular component in an amount of 1 to 10 wt% based on the total weight of the composition. Suitably, the active ingredient can be present in an amount of 1 wt% or more, 2 wt% or more, 3 wt% or more, 4 wt% or more, or 5 wt% or more, and/or 10 wt% or less, 9 wt% or less, 8 wt% or less, 7 wt% or less, or 6 wt% or less, based on the total weight of the composition.

Suitably, the sweetener can comprise, but not be limited to, sucrose, and/or maltitol. The sweetener can be in the form of powder.

Based on the total weight of the composition, the sweetener can be present in an amount of 10 to 40 wt%. Suitably, the sweetener can be present in an amount of 10 wt% or more, 15 wt% or more, 20 wt% or more, and/or 40 wt% or less, 35 wt%, or 30 wt%, based on the total weight of the composition.

In the composition of the present invention, the extragranular component can further comprise other excipients in view of the use of a tablet, for example, disintegrants, flavors, chelating agents, thickening agents, dispersants, stabilizers, suspending agents, adsorbents, granulating agents, preservatives, coloring agents and sweeteners or the like.

In the composition of the present invention, the weight ratio of the intragranular component to the extragranular component can comprise 1.5:1 to 5:1. Suitably, the weight ratio of the intragranular component to the extragranular component can be 1.5:1 or more, 2:1 or more, 2.2:1 or more, 2.4:1 or more, 2.6:1 or more, 2.8:1 or more, 3:1 or more, and/or 5:1 or less, 4.5:1 or less, 4:1 or less, 3.8:1 or less, 3.6:1 or less, 3.4:1 or less, or 3.2:1 or less. For example, the weight ratio of the intragranular component to the extragranular component can be 1.5:1 to 5:1, 2:1 to 4.5:1, or within any range using any of the above ratios as endpoints.

The present invention further refers to a tablet prepared from materials comprising the above composition. In some embodiments, the tablet can be prepared from the above composition. In some embodiments, the tablet can be prepared from materials comprising the above composition and a coating composition. The coating composition can form a coating layer of the tablet.

Suitably, the tablet of the present invention is chewable. As used herein, the term "chewable tablets" are an oral dosage form intended to be chewed and then swallowed by a person rather than swallowed whole. In the present invention, the chewable tablet can become more chewy without disintegration as a person chew it. The chewable tablet can have a mouthfeel like chocolate. The chewiness of the tablet and other sensory attributes can be determined as shown in the Example section below.

The tablet of the present invention can have a weight of 800 mg, 900 mg, 1000 mg, 1100 mg, 1200 mg, 1300 mg, 1400 mg, 1500 mg, 1600 mg, 1700 mg, 1800 mg, 1900 mg, 2000 mg, 2100 mg, 2200 mg, 2300 mg, 2400 mg, or 2500 mg.

The materials comprising the above composition can have a bulk density of 0.5 to 1 g/ml. As use herein, the term "bulk density" refers to the ratio of the mass of an untapped powder sample and its volume including the contribution of the interparticulate void volume. Suitably, the materials comprising the above composition can have a bulk density of 0.5 g/ml or more, 0.6 g/ml or more, or 0.7 g/ml or more, and/or 1 g/ml or less, 0.9 g/ml or less, or 0.8 g/ml or less.

In some embodiments, the above composition can have a bulk density of 0.5 to 1 g/ml. Suitably, the above composition can have a bulk density of 0.5 g/ml or more, 0.6 g/ml or more, or 0.7 g/ml or more, and/or 1 g/ml or less, 0.9 g/ml or less, or 0.8 g/ml or less. The materials comprising the above composition or the above composition with suitable bulk density can be compressed into tablet.

Further, the present invention refers to a method for preparing a chewable tablet from materials comprising the above composition, comprising:
(1) mixing the diluent and the binder by adding the binder into the diluent;
(2) granulating the mixture obtained from Step (1) to form granules;
(3) mixing the extragranular component with the granules obtained in Step (2); and
(4) compressing.

Suitably, the method can further comprise a binder preparation step prior to the above Step (1): mixing the saccharide, the emulsifier and the fat to provide a binder in liquid form. The mixing can be performed under heat. For example, the mixing can be performed at 60 to 120°C, such as 70 to 110°C, 80 to 100°C.

Suitably, the method can further comprise a diluent preparation step prior to the above Step (1): dry mixing the first diluent and optional the second diluent. Suitably, the dry mixing step can be performed with an impeller at about 70-90 RPM (referred to as "Slow impeller" below) for 5-20 seconds.

Suitably, the granulating in Step (2) can be performed by kneading with an impeller at about 70-90 RPM (referred to as "Slow impeller" below) then a chopper at about 1500-1700 RPM (referred to as "Fast chopper" below) for 10-60 seconds.

If more than one emulsifier is added into the composition in Step (3), each emulsifier can be added individually. For example, the Step (3) can comprise: (i) adding the sweetener and kneading with an impeller at about 190-210 RPM (referred to as "Fast impeller") then a chopper at about 1500-1700 RPM (referred to as "Fast chopper" below) for 10-60 seconds; and/or (ii) adding each of the emulsifier individually.

Suitably, the method of the present invention can further comprise: (5) applying a coating layer to the tablet.

### EXAMPLES

The following examples are provided to further illustrate the present invention, but should not be construed to limit the present invention to the details of the examples. All parts and percentages in the following examples are by weight, unless otherwise stated.

### Example 1:

The tablets of Example 1 were prepared using the ingredients and their respective amounts listed in Table 1 below, specifically by the steps of: (1) mixing liquid glucose, vegetable fat and ammonium phosphatide under heat at about 90°C, then cooling to room temperature; (2) mixing calcium carbonate in a Saizoner Mixer Granulator (from Sainath Boilers & Pneumatics) at Slow impeller (about 81 RPM) for 10 seconds; (3) adding the mixture obtained from Step (1) into the Saizoner Mixer Granulator, and kneading with Slow impeller (about 81 RPM) and Fast chopper (about 1600 RPM) for 30 seconds; (4) adding sucrose powder into the Saizoner Mixer Granulator and kneading with Fast impeller (about 200 RPM) and Fast chopper (about 1600 RPM) for 30 seconds (5) passing the particles obtained from Step (4) through a Vibrosifter (from Shiv Pharma Engineering) with 18 # Mesh (6) milling the retained particles from Step (5) in a Mixer Grinder (from Ultra) and collect both the granules in polybag; (7) adding Cabosil (silicon dioxide) and blending for 2 minutes followed by adding magnesium stearate and Lubricating for 2 minutes and (8) transferring the blend obtained in Step (7) to a Compression Machine of D Tooling-8 stations (from Remik), with a punch having 16 mm round shaped deep concave and 9.5 mm thickness. The tablets of Example 1 have a thickness of 9.5 mm

**Table 1.**

| **Batch size: 1000 tablets** | | | | |
|---|---|---|---|---|
| | **Ingredients** | **Supplier** | **% w/w** | **mg/tablet** |
| **1** | Calcium Carbonate | Sudeep Pharma | 37.5 | 750.00 |
| **2** | Ammonium Phosphatide | AMP 4455 from Palsgaard | 0.25 | 5.00 |
| **3** | Liquid Glucose | Cargill | 30 | 600.00 |
| **4** | Vegetable Fat | KG 401 from AAkamani | 2.5 | 50.00 |
| **5** | Sucrose Powder | MB Sugars | 28.05 | 561.00 |
| **6** | Magnesium Stearate | Prachin Chemicals | 1.4 | 28.00 |
| **7** | Silicon Dioxide | Cabosil M5P from Cabot Sanmar | 0.3 | 6.00 |
| **Tablet Weight** | | | **2000 mg** | |

### Example 2

The tablets of Example 2 were prepared using the ingredients and their respective amounts listed in Table 2 below, specifically by the steps of: (1) melting fructooligosaccharides powder with 20 wt% of purified water (on the basis of the weight of the fructooligosaccharides powder) and then mixing with vegetable fat and ammonium phosphatide under heat at about 90°C, then cooling to room temperature; (2) mixing tricalcium phosphate in a Saizoner Mixer Granulator (from Sainath Boilers & Pneumatics) at Slow impeller (about 81 RPM) for 10 seconds; (3) adding the mixture obtained from Step (1) into the Saizoner Mixer Granulator, and kneading with slow impeller for 40 seconds and Fast chopper (about 1600 RPM) for 40 seconds (4) adding maltitol powder into the Saizoner Mixer Granulator and kneading with Fast impeller (about 200 RPM) and Fast chopper (about 1600 RPM) for 40 seconds; (5) passing the particles obtained from Step (4) through a Vibrosifter (from Shiv Pharma Engineering) with 20 # mesh (6) milling the retained particles from Step (5) in a Mixer Grinder (from Ultra) and collecting both the granules in a polybag; (7) adding Cabosil (silicon dioxide) and blending for 2mins followed by adding magnesium stearate and lubricating for 2 minutes; and (8) transferring the blend obtained in Step (7) to a Compression Machine of D Tooling-8 stations (from Remik), with a punch having 16 mm round shaped deep concave and 9.5 mm thickness.

**Table 2.**

| **Batch size: 1000 tablets** | | | | |
|---|---|---|---|---|
| | **Ingredients** | **Supplier** | **% w/w** | **mg/tablet** |
| **1** | Tricalcium Phosphate | Sudeep Pharma | 39.67 | 833.00 |
| **2** | Ammonium Phosphatide | AMP 4455 from Palsgaard | 0.33 | 7.00 |
| **3** | Fructooligosaccharides Powder (FOS P95) | Tata Chemicals Ltd | 38.10 | 800.00 |
| **4** | Vegetable Fat | KG 401 from AAkamani | 2.86 | 60.00 |
| **5** | Maltitol Powder | MB Sugars | 17.24 | 362.00 |
| **6** | Magnesium Stearate | Prachin Chemicals | 1.33 | 28.00 |
| **7** | Silicon Dioxide | Cabosil M5P from Cabot Sanmar | 0.48 | 10.00 |
| **Tablet Weight** | | | **2100 mg** | |

### Example 3

The tablets of Example 3 were prepared using the ingredients and their respective amounts listed in Table 3 below, specifically by the steps of: (1) mixing liquid glucose, vegetable fat and ammonium phosphatide under heat at about 90°C, then cooling to room temperature; (2) mixing tricalcium phosphate in a Saizoner Mixer Granulator (from Sainath Boilers & Pneumatics) at Slow impeller (about 81 RPM) for 10 seconds; (3) adding the mixture obtained from Step (1) into the Saizoner Mixer Granulator, and kneading with Slow impeller (about 81 RPM) for 40 seconds and Fast chopper (about 1600 RPM) for 40 seconds); (4) adding sucrose powder into the Saizoner Mixer Granulator and kneading with Fast impeller (about 200 RPM) and Fast chopper (about 1600 RPM) for 40 seconds; (5) passing the particles obtained from Step (4) through a Vibrosifter (from Shiv Pharma Engineering) with 20# mesh; (6) milling the retained particles from Step (5) in a Mixer Grinder (from Ultra) and collect both the granules in a polybag; (7) adding silicon dioxide, Vitamin D2 and Zinc Citrate and blending for 5 mins followed by adding magnesium stearate, and lubricating for 2 minutes and (8) transferring the blend obtained in Step (7) to a Compression Machine of D Tooling-8 stations (from Remik), with a punch having 16 mm round shaped deep concave and 9.5 mm thickness.

**Table 3.**

| **Batch size: 1000 tablets** | | | | |
|---|---|---|---|---|
| | **Ingredients** | **Supplier** | **% w/w** | **mg/tablet** |
| **1** | Tricalcium Phosphate | Sudeep Pharma | 39.67 | 833.00 |
| **2** | Ammonium Phosphatide | AMP 4455 from Palsgaard | 0.38 | 8.00 |
| **3** | Liquid Glucose | Cargill | 33.33 | 700.00 |
| **4** | Vegetable Fat | KG 401 from AAkamani | 2.86 | 60.00 |
| **5** | Sucrose Powder | MB Sugars | 19.04 | 399.79 |
| **6** | Magnesium Stearate | Prachin Chemicals | 1.67 | 35.00 |
| **7** | Silicon Dioxide | Cabosil M5P from Cabot Sanmar | 0.48 | 10.00 |
| **8** | Vitamin D2 | Piramal | 0.13 | 2.66 |
| **9** | Zinc Citrate | Aditya Chemicals | 2.45 | 51.55 |
| **Tablet Weight** | | | **2100 mg** | |

### Example 4

The tablets of Example 4 were prepared using the ingredients and their respective amounts listed in Table 4 below, specifically by the steps of: (1) mixing liquid glucose, vegetable fat and ammonium phosphatide under heat at about 90°C, then cooling to room temperature; (2) mixing tricalcium phosphate in a Saizoner Mixer Granulator (from Sainath Boilers & Pneumatics) at Slow impeller (about 81 RPM) for 10 seconds; (3) adding the mixture obtained from Step (1) into the Saizoner Mixer Granulator, and kneading with Slow impeller (about 81 RPM) for 40 seconds and Fast chopper (about 1600 RPM) for 40 seconds (4) adding sucrose powder into the Saizoner Mixer Granulator and kneading with Fast impeller (about 200 RPM) and Fast chopper (about 1600 RPM) for 40 seconds; (5) passing the particles obtained from Step (4) through a Vibrosifter (from Shiv Pharma Engineering) with 20# mesh (6) milling the retained particles from Step (5) in a Mixer Grinder (from Ultra) and collect both the granules in a polybag; (7) adding, silicon dioxide, Vitamin D2, zinc citrate and UCII and blending for 5 minutes followed by adding Magnesium stearate for 2 mins and (8) transferring the compositions obtained in Step (7) to a Compression Machine of D Tooling-8 stations (from Remik), with a punch having 16 mm round shaped deep concave and 9.5 mm thickness.

**Table 4.**

| **Batch size: 1000 tablets** | | | | |
|---|---|---|---|---|
| | **Ingredients** | **Supplier** | **% w/w** | **mg/tablet** |
| **1** | Tricalcium Phosphate | Sudeep Pharma | 39.67 | 833.00 |
| **2** | Ammonium Phosphatide | AMP 4455 from Palsgaard | 0.38 | 8.00 |
| **3** | Liquid Glucose | Cargill | 33.33 | 700.00 |
| **4** | Vegetable Fat | KG 401 from AAkamani | 2.86 | 60.00 |
| **5** | Sucrose Powder | MB Sugars | 18.40 | 386.46 |
| **6** | Magnesium Stearate | Prachin Chemicals | 1.67 | 35.00 |
| **7** | Silicon Dioxide | Cabosil M5P from Cabot Sanmar | 0.48 | 10.00 |
| **8** | Vitamin D2 | Piramal | 0.13 | 2.66 |
| **9** | Zinc Citrate | Aditya Chemicals | 2.45 | 51.55 |
| **10** | UCII | Lonza | 0.63 | 13.33 |
| **Tablet Weight** | | | **2100 mg** | |

### Example 5

The tablets of Example 5 were prepared using the ingredients and their respective amounts listed in Table 5 below, specifically by the steps of: (1) mixing liquid glucose, vegetable fat and ammonium phosphatide under heat at about 90°C, then cooling to room temperature; (2) mixing tricalcium phosphate in a Saizoner Mixer Granulator (from Sainath Boilers & Pneumatics) at Slow impeller (about 81 RPM) for 10 seconds; (3) adding the mixture obtained from Step (1) into the Saizoner Mixer Granulator, and kneading with Slow impeller (about 81 RPM) for 40 seconds and Fast chopper (about 1600 RPM) for 40 seconds; (4) adding sucrose powder into the Saizoner Mixer Granulator and kneading with Fast impeller (about 200 RPM) and Fast chopper (about 1600 RPM) for 40 seconds; (5) passing the particles obtained from Step (4) through a Vibrosifter (from Shiv Pharma Engineering) with 20# mesh; (6) milling the retained particles from Step (5) in a Mixer Grinder (from Ultra) and collect both the granules in a polybag; (7) adding, silicon dioxide, Melatonin and L Theanine and blending for 5 mins followed by adding magnesium stearate and lubricating for 2 minutes and (8) transferring the compositions obtained in Step (7) to a Compression Machine of D Tooling-8 stations (from Remik), with a punch having 16 x 6.95 mm oval shaped dimension.

**Table 5.**

| **Batch size: 1000 tablets** | | | | |
|---|---|---|---|---|
| | **Ingredients** | **Supplier** | **% w/w** | **mg/tablet** |
| **1** | Tricalcium Phosphate | Sudeep Pharma | 34.67 | 416.00 |
| **2** | Ammonium Phosphatide | AMP 4455 from Palsgaard | 0.33 | 4.00 |
| **3** | Liquid Glucose | Cargill | 29.17 | 350.00 |
| **4** | Vegetable Fat | KG 401 from AAkamani | 2.50 | 30.00 |
| **5** | Sucrose Powder | MB Sugars | 23.17 | 278.00 |
| **6** | Magnesium Stearate | Prachin Chemicals | 1.17 | 14.00 |
| **7** | Silicon Dioxide | Cabosil M5P from Cabot Sanmar | 0.42 | 5.00 |
| **8** | Melatonin | Luotian Xinpusheng Pharmaceuticals | 0.25 | 3.00 |
| **9** | L- Theanine | Alspure | 8.33 | 100.00 |
| **Tablet Weight** | | | **1200 mg** | |

The tablets of Example 1-5 were tested for sensory attributes and stability.

### 1- Sensory attributes

The tablets of Examples 1-5 were tested for sensory attributes initially and after storage in a warm and humid environment. According to the tests, the tablets of Examples 1-5 have optimum chewiness and overall acceptability both initially and after storage in a warm and humid environment. The test results of Example 1 are shown as an exemplary in Tables 6-7 below.

**Table 6**

| **Initial sample** | |
|---|---|
| Attribute | Description |
| Appearance | White colored soft chewable tablet |
| Aroma | Sweet note |
| Taste | Sweet, lite chalky |
| Texture | chewiness was optimum |
| Aftertaste | After taste was found little chalky |
| Overall Acceptability | Acceptable with little chalky |

**Table 7**

| | **40°C/75%RH** | **50°C** | |
|---|---|---|---|
| | **1 Month** | **15 Days** | **1 Month** |
| Attribute | Description | Description | Description |
| Appearance | Off white colored soft chewable tablet | Off white colored Soft chewable tablet | Light brown colored soft chewable tablet |
| Aroma | Sweet note | Sweet note | Sweet note |
| Taste | Sweet, little chalky | Sweet, little chalky | Sweet, little chalky |
| Texture | chewiness was optimum | Chewiness was optimum | Chewiness was optimum |
| Aftertaste | After taste was found little chalky | After taste was found little chalky | After taste was found little chalky |
| Overall Acceptability | Acceptable with little chalky | Acceptable | Acceptable with little chalky |

### 2-Stablity

The tablets of Examples 1-5 were evaluated in terms of various parameters both before (Initially) and after storage in a warm and humid environment for stability assessment.

Tables 8-9 show the stability test results for the tablets of Example 1.

**Table 8**

| **Test Parameters** | **Specification** | **Initial** | **40°C/75%RH** |
|---|---|---|---|
| | | | **1 Month** |
| Description | White Colored Soft chewable tablet | White colored soft chewable tablet | Off white colored soft chewable tablet |
| Loss on Drying (Halogen Moisture Analyzer) | < 5 wt% | 3.97 wt% | 3.73 wt% |
| Average Weight | 2.00 g ± 5.0 wt% (1.99 g to 2.10 g) | 2.05 g | 2.04 g |
| Assay of Calcium carbonate by titration method | 90 -110 wt% | 100.08 wt% | 99.00 wt% |

**Table 9**

| **Test Parameters** | **Specification** | **Initial** | **50°C/** | |
|---|---|---|---|---|
| | | | **15 Days** | **1 M** |
| Description | White Coloured Soft chewable tablet | White Coloured Soft chewable tablet | Off White Coloured soft chewable Tablet | Light Brown Coloured soft chewable tablet |
| Loss on Drying (Halogen Moisture analyser) | < 5 wt% | 3.97% | 3.42 % | 3.50% |
| Average Weight | 2.00 g±5.0 wt% (1.99 to 2.10 g) | 2.05 g | 2.04 g | 2.05 g |
| Assay of Calcium carbonate by titration method | 90 -110 wt% | 100.08 wt% | 99.00 wt% | 99.01 wt% |

### Comparative Example 1

The products of Comparative Example 1 were prepared using the ingredients and their respective amounts listed in Table 10 below, specifically by the steps similar to those of the previous examples, comprising: (1) mixing liquid glucose, sucrose and HOSO powder under heat at about 90°C, then cooling to room temperature; (2) dispersing starch slurry into the mixture obtained from Step (1); and (3) granulating Calcium Carbonate in a Saizoner Mixer Granulator (from Sainath Boilers & Pneumatics) with the mixture obtained from Step (2).

**Table 10**

| | **Ingredients** | **Supplier** | **% w/w** | **mg** |
|---|---|---|---|---|
| **1** | Calcium Carbonate | Sudeep Pharma | 36.34 | 750.00 |
| **2** | Liquid Glucose | Cargill | 30.28 | 625.00 |
| **3** | Sucrose | MB Sugar | 30.28 | 625.00 |
| **4** | HOSO Powder | Dry tech | 1.65 | 34.00 |
| **5** | Starch | Universal starch | 1.45 | 30.00 |
| **Total Weight** | | | **2064 mg** | |

The granules obtained did not have any chewy mouthfeel, thus were not compressed.

### Comparative Example 2

The tablets of Comparative Example 2 were prepared using the ingredients and their respective amounts listed in Table 11 below, specifically by the steps of: (1) mixing liquid glucose, sucrose and butter under heat at about 90°C, then cooling to room temperature; (2) mixing calcium carbonate and sugar powder in a Saizoner Mixer Granulator (from Sainath Boilers & Pneumatics); (3) dispersing starch slurry into the mixture obtained from Step (1); (4) granulating calcium carbonate and sugar powder in a Saizoner Mixer Granulator (from Sainath Boilers & Pneumatics) with the mixture obtained from Step (3); (5) drying the granules obtained from Step (4) in a fluid bed processor and screening; (6) adding guargum, lubritab and xanthan gum and blending; and (7) compressing.

**Table 11**

| **Batch size: 1000 tablets** | | | | |
|---|---|---|---|---|
| | **Ingredients** | **Supplier** | **% w/w** | **mg/tablet** |
| **1** | Calcium Carbonate | Sudeep Pharma | 35.48 | 750.00 |
| **2** | Sugar Powder | MB Sugar | 28.38 | 600.00 |
| **3** | Liquid Glucose | Cargill | 14.19 | 300.00 |
| **4** | Sucrose | MB Sugar | 14.19 | 300.00 |
| **5** | Butter | Amul | 1.61 | 34.00 |
| **6** | Starch | Universal starch | 1.42 | 30.00 |
| **7** | A Guargum/ | Vasundhara | 4.73 | 100.00 |
| | B Lubritab | SA Pharma | | |
| | C Xanthan gum | CP Kelco | | |
| **Tablet Weight** | | | **2114 mg** | |

The compressed tablets had initial chewy mouthfeel, followed by sticky and slippery feel which is not acceptable.

### Comparative Example 3

The products of Comparative Example 3 were prepared using the ingredients and their respective amounts listed in Table 12 below, specifically by the steps of: (1) mixing liquid glucose and sucrose under heat at about 90°C, then cooling to room temperature; (2) mixing calcium carbonate, sunflower oil and sugar powder in a Saizoner Mixer Granulator (from Sainath Boilers & Pneumatics); (3) dispersing starch slurry with Tween 80 into the mixture obtained from Step (1); (4) granulating the mixture of Step (2) in a Saizoner Mixer Granulator (from Sainath Boilers & Pneumatics) with the mixture obtained from Step (3); and (5) drying the granules obtained from Step (4) in a hot air oven and screening.

**Table 12**

| | **Ingredients** | **Supplier** | **% w/w** | **mg** |
|---|---|---|---|---|
| **1** | Calcium Carbonate | Sudeep Pharma | 41.16 | 750.00 |
| | Sugar Powder | MB Sugar | 21.95 | 400.00 |
| **2** | Liquid Glucose | Cargill | 21.95 | 400.00 |
| **3** | Sucrose | MB Sugar | 8.23 | 150.00 |
| **4** | Sunflower Oil | AOS Products | 3.84 | 70.00 |
| **5** | Starch | **Universal Starch** | 2.74 | 50.00 |
| **6** | Tween 80 | **Croda** | 0.10 | 2.00 |
| **Total Weight** | | | **1822 mg** | |

The granules were not properly formed (mostly threads), thus were not compressed.

### Comparative Example 4

The tablets of Comparative Example 4 were prepared using the ingredients and their respective amounts listed in Table 13 below, specifically by the steps of: (1) mixing liquid glucose, sucrose, gum acacia and coca butter under heat at about 90°C, then cooling to room temperature; (2) mixing calcium carbonate and sugar powder in a Saizoner Mixer Granulator (from Sainath Boilers & Pneumatics) with purified water; (3) dispersing starch slurry into the mixture obtained from Step (1); (4) granulating in a Saizoner Mixer Granulator (from Sainath Boilers & Pneumatics) with the mixture obtained from Step (3); (5) drying the granules obtained from Step (4) in a fluid bed processor and screening; and (6) compressing.

**Table 14**

| **Batch size: 1000 tablets** | | | | |
|---|---|---|---|---|
| | **Ingredients** | **Supplier** | **% w/w** | **mg/tablet** |
| **1** | Calcium Carbonate | Sudeep Pharma | 41.32 | 750.00 |
| **2** | Sugar Powder | MB Sugar | 16.53 | 300.00 |
| **3** | Liquid Glucose | Cargill | 16.53 | 300.00 |
| **4** | Sucrose | MB Sugar | 16.53 | 300.00 |
| **5** | Cocoa Butter | AOS Products | 1.93 | 35.00 |
| **6** | Starch | Universal Starcj | 1.65 | 30.00 |
| **7** | Gum acacia | Alland & Robert | 5.51 | 100.00 |
| **Tablet Weight** | | | **1815** | |

The compressed tablets did not have any chewy mouthfeel.

### Comparative Example 5

The tablets of Comparative Example 5 were prepared using the ingredients and their respective amounts listed in Table 14 below, specifically by the steps of: (1) mixing liquid glucose, sorbitol and cocoa butter under heat at about 90°C, then cooling to room temperature; (2) mixing calcium carbonate and sugar powder A in a Saizoner Mixer Granulator (from Sainath Boilers & Pneumatics) with purified water; (3) granulating the mixture of Step (2) in a Saizoner Mixer Granulator (from Sainath Boilers & Pneumatics) with the mixture obtained from Step (1); (4) mixing the mixture obtained from Step (3) with sugar powder B, vanilla flavour, plyox WSR and magnesium stearate; and (5) compressing.

**Table 14**

| **Batch size: 1000 tablets** | | | | |
|---|---|---|---|---|
| | **Ingredients** | **Supplier** | **% w/w** | **mg/tablet** |
| **1** | Calcium Carbonate | Sudeep Pharma | 39.55 | 750.00 |
| **2** | Sugar Powder A | MB Sugar | 15.82 | 300.00 |
| **3** | Liquid Glucose | Cargill | 26.37 | 500.00 |
| **4** | Cocoa Butter | AOS Products | 1.85 | 35.00 |
| **5** | Sorbitol 70% Solution | Sankalp | 3.69 | 70.00 |
| **6** | Sugar Powder B | MB Sugar | 10.55 | 200.00 |
| **7** | Polyox WSR | Colorcon | 0.79 | 15.00 |
| **8** | Vanilla Flavour | Taste Master | 0.39 | 7.50 |
| **9** | Magnesium Stearate | Prachin chemicals | 0.99 | 18.77 |
| **Tablet Weight** | | | **1896.27** | |

The compressed tablets did not have good chewable mouthfeel.

Although the particular aspects of the present invention have been illustrated and described, it is obvious to persons skilled in the art that many other variations and modifications can be made without departing the spirit and scope of the present invention. Thus, the accompanying claims are intended to encompass all of these variations and modifications falling within the scope of the present invention.

## Claims

**1.** A composition, comprising an intragranular component and an extragranular component, the intragranular component comprising a diluent and a binder, wherein the diluent comprises 40 wt% or more of a first diluent based on the total weight of the diluent, the first diluent comprises one or more selected from the group consisting of: calcium carbonate, tricalcium phosphate, magnesium carbonate, and mixtures thereof, and the binder comprises a saccharide, an emulsifier and a fat.

**2.** The composition of claim 1, wherein the saccharide comprises one or more selected from the group consisting of: liquid glucose, sorbitol solution, maltitol syrup, corn syrup, maple syrup, brown rice syrup, liquid maltrin, a solution of fructo oligosaccharide powder, and mixtures thereof.

**3.** The composition of claim 1 or 2, wherein the emulsifier comprises one or more selected from the group consisting of: ammonium phosphatide, a lecithin, a polyglycerol polyricinoleate, an acetate of monodiglyceride, an acetate of diglyceride, and mixtures thereof.

**4.** The composition of any one of claims 1-3, wherein the fat comprises one or more selected from the group consisting of: a vegetable fat, a hydrogenated fat/oil, milk butter, and mixtures thereof.

**5.** The composition of any one of claims 1-4, wherein the binder comprises 85 wt% or more of the saccharide based on the total weight of the binder.

**6.** The composition of any one of claims 1-5, wherein the binder comprises 5 wt% or less of the emulsifier based on the total weight of the binder.

**7.** The composition of any one of claims 1-6, wherein the binder comprises 10 wt% or less of the fat based on the total weight of the binder

**9.** The composition of any one of claims 1-8, wherein the binder comprises 90-94 wt% of the saccharide, 0.5-2 wt% of the emulsifier, and 5-10 wt% of the fat, based on the total weight of the binder.

**10.** The composition of any one of claims 1-9, wherein the diluent further comprises a second diluent selected from the group consisting of: sucrose, maltitol, starch, mixtures thereof.

**11.** The composition of any one of claims 1-10, wherein the weight ratio of the diluent to the binder comprises 2:1 to 0.6:1.

**12.** The composition of any one of claims 1-11, wherein the extragranular component comprises a lubricant, a sweetener, and/or an active ingredient.

**13.** The composition of claim 12, wherein the lubricant comprises magnesium stearate, and/or silicon dioxide.

**14.** The composition of claim 12 or 13, wherein the active ingredient comprises one of more of selected from the group consisting of: calcium, magnesium, vitamin D2, zinc citrate, UCII, vitamin B, melatonin, L-theanine, probiotics, pepper, ginger, cumin, black salt, and mixtures thereof.

**15.** The composition of any one of claims 12-14, wherein the active ingredient comprises an active pharmaceutical ingredient.

**16.** The composition of any one of claims 1-15, wherein the weight ratio of the intragranular component to the extragranular component comprises 1.5:1 to 5:1.

**17.** A tablet prepared from materials comprising the composition of any one of claims 1-16.

**18.** The tablet of claim 17, wherein the tablet is chewable.

**19.** The tablet of claim 17 or 18, wherein the tablet has a weight of 800 mg, 900 mg, 1000 mg, 1100 mg, 1200 mg, 1300 mg, 1400 mg, 1500 mg, 1600 mg, 1700 mg, 1800 mg, 1900 mg, 2000 mg, 2100 mg, 2200 mg, 2300 mg, 2400 mg, or 2500 mg.

**20.** The tablet of any one of claims 16-19, wherein the materials blend has a bulk density of 0.5 to 1 g/ml.

**21.** A method for preparing a tablet from materials comprising the composition of any one of claims 1-15, comprising:
(1) mixing the diluent and the binder by adding the binder into the diluent;
(2) granulating the mixture obtained from Step (1) to form granules;
(3) mixing the extragranular component with the granules obtained in Step (2); and
(4) compressing.

**22.** The method of claim 21, wherein the tablet is chewable.
